# EUROPÄISCHE PATENTSCHRIFT

(11) **EP 0 535 484 B1**
(45) Veröffentlichungstag und Bekanntmachung des Hinweises auf die Patenterteilung: **03.01.1996**
(21) Anmeldenummer: 92116089.1
(22) Anmeldetag: 21.09.1992
(51) Int. Cl.: C07C 209/28, C07C 211/55

(54) **Verfahren zur Herstellung von Diphenylaminen**
Process for the preparation of diphenylamines
Procédé pour la préparation de diphénylamines

(30) Priorität: 04.10.1991 DE 4132945
(43) Veröffentlichungstag der Anmeldung: 07.04.1993
(73) Patentinhaber: BAYER AG, D-51368 Leverkusen (DE)
(72) Erfinder: Immel, Otto, Dr., W-4150 Krefeld (DE); Darsow, Gerhard, Dr., W-4150 Krefeld (DE); Buysch, Hans-Josef, Dr., W-4150 Krefeld (DE)

(56) Entgegenhaltungen:
- EP-A- 0 208 933
- EP-A- 0 325 132
- FR-A- 2 561 238
- US-A- 3 219 704

## Beschreibung

Die Erfindung betrifft ein Verfahren zur Herstellung von gegebenenfalls substituierten Diphenylaminen durch Umsetzung der zugrundeliegenden Cyclohexanone und Aniline in Gegenwart eines Rhodium enthaltenden Trägerkatalysators.

Zur Herstellung von Diphenylamin und dessen Derivaten ist aus der DE-OS 2 331 878 ein Verfahren bekannt, bei dem man von Iminen, wie N-Cyclohexyliden-anilin und dessen Derivaten, ausgeht und sie in der Gasphase in Gegenwart von Trägerkatalysatoren auf der Basis von Nickel, Platin, Palladium oder Kupfer-Chrom dehydriert.

Weiterhin ist aus DE-OS 2 520 893 bekannt, Diphenylamin durch katalytische Dehydrierung von Verbindungen und/oder Verbindungsgemischen, die aus ganz oder teilweise hydriertem Diphenylamin bestehen, in Gegenwart eines Nickel-Chrom, Aluminium, Kupfer, Mangan und Alkali enthaltenden Dehydierungskatalysators herzustellen. Als solche Verbindungen werden zweikernige aromatische Imine in den Ausführungsbeispielen gezeigt.

Auch die Dehydrierung von Dicyclohexylamin zu Diphenylamin an Edelmetall-Katalysatoren wurde bereits vorgeschlagen (DE-OS 3 801 754).

Die genannten Verfahren erfordern relativ kostspielig herzustellende Ausgangsmaterialien, wie N-Cyclohexyliden-anilin oder Dicyclohexylamin und sind für ein industriell durchzuführendes Verfahren verbesserungsbedürftig.

Aus US 3 219 704 ist ein diskontinuierliches Rührkesselverfahren zur Herstellung von Diarylaminen aus cyclischen Ketonen und Aminoverbindungen wie Anilin bekannt, bei dem in einer ein- bis sechsstündigen Reaktion unter Rühren der Ausgangsverbindungen ein pulverförmiger Trägerkatalysator (Pd oder Pt auf A-Kohle) in Suspension zum Einsatz kommt.

Ein weiteres diskontinuierliches Autoklaven-Verfahren in der Flüssigphase zur Herstellung von Diphenylaminen oder N,N'-Diphenyl-phenylendiaminen ist aus FR-A 2 561 238 bekannt. Hierin werden Derivate von Anilin bzw. Phenylendiamin mit überschüssigem Phenol umgesetzt; Cyclohexanon wird nicht in molaren Mengen eingesetzt, sondern nur in den geringen Mengen, wie es sich durch unvermeidliche Kernhydrierung aus Phenol bildet und in das Reaktionsgemisch zurückgeführt wird. Zur Umsetzung wird ein Pd-Katalysator benutzt.

Die Erfindung betrifft ein Verfahren zur Herstellung von Diphenylamin der Formel 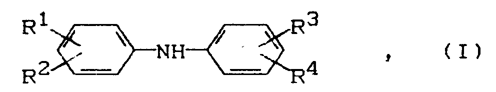
in der
- R¹, R², R³ und R⁴: unabhängig voneinander Wasserstoff C₁-C₄-Alkyl oder C₁-C₄-Alkoxy bedeuten,
das dadurch gekennzeichnet ist, daß Aniline der Formel 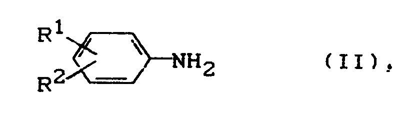
mit Cyclohexanonen der Formel 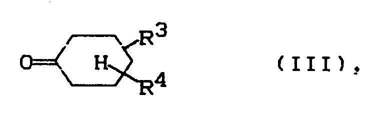
die sich im Gemisch mit recyclisierten Reaktionsprodukten befinden können, wobei in den Formeln die Reste R¹ - R⁴ die obige Bedeutung haben, und wobei Anilin:Cyclohexanon im molaren Verhaltnis von 1:10-10:1, bevorzugt 1:2-6:1, vorliegen, in verdampfter Form an einem 0,05-5 Gew.-%, bevorzugt 0,05-4 Gew.-%, besonders bevorzugt 0,1-3 Gew.-%, bezogen auf das Gesamtgewicht des Katalysators, Rhodium oder eine Kombination von Rhodium mit mindestens einem Platinmetall aus der Gruppe Palladium, Platin, Ruthenium und Iridium enthaltenden Trägerkatalysator, der weiterhin 1 bis 12 Gew.-%, bezogen auf das Katalysatorgewicht, eines oder mehrerer Alkalimetallhydroxide und/oder Alkalimetallsulfate enthält, wobei die Hydroxidanteile und die Sulfatanteile jeweils höchstens 6 Gew.-% betragen, bei 200-450°C und 0,1-20 bar umgesetzt werden.

Im erfindungsgemäßen Verfahren vollzieht sich offenbar zunächst eine Kondensation von Anilin und Cyclohexanon, von der angenommen werden kann, daß sie unter Wasseraustritt zum N-Cyclohexyliden-anilin führt, welches dann zum Diphenylamin dehydriert wird. Der erfolgreiche Verlauf des erfindungsgemäßen Verfahrens ist in verschiedener Weise überraschend. So ist bekannt, daß die Bildung des N-Cyclohexyliden-anilins eine Gleichgewichtsreaktion darstellt, die stets nennenswerte Teile der Ausgangsprodukte zuläßt. So mußte man stets mit der Gefahr rechnen, daß das Cyclohexanon zu in diesem Verfahren wertlosem Phenol aromatisiert wird. Des weiteren wäre zu erwarten gewesen, daß die intermediäre Bildung des N-Cyclohexyliden-anilins nicht sehr begünstigt ist, da zu seiner Bildung ausgesprochen saure Katalysatoren erforderlich sind.

Das erfindungsgemäße Verfahren birgt insoweit ein weiteres Überraschungsmoment, als nicht nur, wie oben geschildert wurde, die durchaus denkbare Bildung von Phenol aus dem Cyclohexanon unterbleibt, sondern sogar zugesetztes Phenol im Sinne von Cyclohexanon reagiert, und damit im Sinne des oben geschilderten Denkmodells sogar eine Umwandlung von Phenol in Cyclohexanon stattfinden muß. Die Menge dieses zugesetzten Phenols, das zur Vermeidung von unnötigen Produktgemischen das gleiche Substitutionsmuster wie das eingesetzte Cyclohexanon haben soll, beträgt 0-20 mol-%, bezogen auf die Gesamtmenge des Gemisches aus Cyclohexanon und Phenol.

Der erfindungsgemäß einzusetzende Katalysator enthält Rhodium oder eine Kombination von Rhodium mit einem anderen Platinmetall aus der Gruppe Palladium, Platin, Ruthenium und Iridium. Die Edelmetalle liegen in einer Gesamtmenge von 0,05-5 Gew.-%, bevorzugt 0,05-4 Gew.-%, besonders bevorzugt 0,1-3 Gew.-% vor, bezogen auf das Gesamtgewicht des Katalysators.

In bevorzugter Weise enthält der erfindungsgemäß einzusetzende Katalysator eine solche Kombination von Rhodium mit mindestens einem der genannten anderen Platinmetalle, in der Rhodium in einer Menge von 10 - 90 % des Gesamtgewichts aller Edelmetalle vorliegt. In besonders bevorzugter Weise wird Rhodium mit Palladium oder Platin oder einem Gemisch aus Palladium und Platin kombiniert. In ganz besonders bevorzugter Weise wird zur Kombination mit dem Rhodium Palladium oder Platin allein eingesetzt. In einer solchen Kombination beträgt der Anteil des Rhodiums 10-90 %, bevorzugt 15-80 %, besonders bevorzugt 20-70 % des Gesamtgewichts aller Edelmetalle.

Der einzusetzende Katalysator enthält weiterhin 1-12 Gew.-%, bevorzugt 2-10 Gew.-%, bezogen auf das Katalysatorgewicht, eines oder mehrerer Alkalimetallhydroxide und/oder Alkalimetallsulfate, wobei die Hydroxidanteile und die Sulfatanteile jeweils höchstens 6 Gew.-%, bevorzugt jeweils höchstens 5 Gew.-% betragen. Beispiele für solche Hydroxide und Sulfate sind: Lithiumhydroxid, Natriumhydroxid, Kaliumhydroxid, Rubidiumhydroxid, Caesiumhydroxid, bevorzugt Lithiumhydroxid, Natriumhydroxid, Kaliumhydroxid, besonders bevorzugt Natriumhydroxid oder Kaliumhydroxid; Lithiumsulfat, Natriumsulfat, Kaliumsulfat, Rubidiumsulfat, Caesiumsulfat, bevorzugt Lithiumsulfat, Natriumsulfat, Kaliumsulfat, besonders bevorzugt Natriumsulfat oder Kaliumsulfat.

Die genannten Bestandteile der einzusetzenden Katalysatoren sind auf einem Träger angeordnet. Beispiele für solche Träger sind Aluminiumoxid, Aluminiumspinell, Aktivkohle, Kieselgel, Bentonit, Bimsstein, Zirkonoxid, Titanoxid, Zinkoxid, Magnesiumoxid sowie Oxide der seltenen Erden.

Die genannten Bestandteile der einzusetzenden Katalysatoren sind bevorzugt auf einem Träger aus Aluminiumoxid oder einem Aluminium-Spinell, angebracht, besonders bevorzugt auf einem solchen Aluminiumoxid oder Aluminium-Spinell, das mit Chrom und Mangan behandelt worden ist. Als Aluminiumoxid kommen insbesondere die α- und die γ-Modifikation infrage. Aluminium-Spinelle sind Verbindungen der Formel

Me(II)Al₂O₄ bzw. Me(I)AlO₂,

in denen Me(II) ein zweiwertiges Metallkation des Eisens, Zinks, Nickels, Kupfers, Kobalts, Cadmiums, Magnesiums oder anderer, bevorzugt des Magnesiums, und Me(I) ein einwertiges Kation, beispielsweise Lithium (Lithium-Aluminium-Spinell), ist. Das Aluminium in den Spinellen kann teilweise durch dreiwertiges Eisen, Chrom oder Mangan ersetzt sein. In bevorzugter Weise wird Al₂O₃, besonders bevorzugt das γ-Al₂O₃, eingesetzt. Ein solcher Träger weist in der besonders bevorzugten Weise einen Gehalt an Chrom und Mangan von zusammen etwa 0,05-8 Gew.-%, bevorzugt 0,2-5 Gew.-%, bezogen auf das Gesamtgewicht des Katalysators, auf. Das Gewichtsverhältnis von Chrom und Mangan beträgt 5:1-1:5, bevorzugt 2:1-1:2. Solche mit Chrom und Mangan behandelten Träger sind aus EP 208 933 bekannt.

Zur Herstellung der beschriebenen Katalysatoren kann in bevorzugter Weise so vorgangen werden, daß auf ein Al₂O₃ in Form von Strangpreßlingen, Pillen oder Kugeln mit Abmessungen von etwa 2-10 mm Verbindungen des Chroms und Mangans aufgebracht werden, der so beaufschlagte Träger auf höhere Temperatur erhitzt wird, anschließend getrennt die Edelmetalle und eines oder mehrere der Alkalihydroxide und/oder eines oder mehrere der Alkalisulfate aufgetragen werden; nach jedem Auftragen wird eine Trocknung vorgenommen, im allgemeinen bei 100-140°C bei vermindertem bis normalem Druck, beispielsweise 1-1000 mbar, bevorzugt 10-500 mbar, beispielsweise im Wasserstrahlvakuum.

Das Aufbringen des Chroms und Mangans auf den Katalysatorträger kann beispielsweise durch gemeinsames Ausfällen eines Mangan-Chrom-Hydroxid-Gemisches aus einer Chrom- und Mangansalz-Lösung mit Alkalilauge oder Ammoniak und anschließendes Auswaschen der löslichen Anteile mit Wasser erfolgen. Als Chrom- und Mangansalze kommen insbesondere die Sulfate, Chloride, Acetate und/oder Nitrate der genannten Elemente in Betracht, Die Abscheidung des Chroms und Mangans auf dem Katalysatorträger kann auch als Ammonium-Mangan-Chromat oder Ammonium-Alkali-Mangan-Chromat aus einer Lösung von Mangan(II)-Salzen und Ammoniumbichromat mittels Ammoniak und/oder basischer Alkaliverbindungen erfolgen. Besonders gleichmäßige und festhaftende Abscheidungen erhält man, wenn die Basenzugabe langsam und gleichmäßig unter Vermeidung größerer Konzentrationsunterschiede erfolgt.

Hierzu kann beispielsweise die Fällung mittels Harnstoff unter hydrolysierenden Bedingungen vorgenommen werden, wodurch die Bedingungen der langsamen Basenzugabe besonders gut gewährleistet sind.

Nach dem Aufbringen der Chrom- und Manganverbindungen und der beschriebenen Ausfällung wird der so beaufschlagte Katalysatorträger frei von löslichen Verbindungen gewaschen, bevor er auf höhere Temperaturen (etwa 200-450°C, bevorzugt 250-350°C) erhitzt wird. Nach dieser Temperung ist der mit Chrom und Mangan beaufschlagte Träger bereit zur Tränkung mit den übrigen genannten Katalysatorbestandteilen.

Die Tränkung des Katalysators mit den Edelmetallen beziehungsweise mit Alkalihydroxid und/oder Alkalisulfat (jeweils eines oder mehrere von diesen) erfolgt getrennt. Hierbei kann so vorgangen werden, daß zunächst die Edelmetalle, beispielsweise in Form wäßriger Lösungen ihrer Chloride, Nitrate, Acetate oder anderer geeigneter Salze auf den Träger aufgetränkt werden, wobei nach der Trocknung eine weitere Tränkung mit einer Lösung von Alkalihydroxid und/oder Alkalimetallsulfat erfolgt. Bei dieser Behandlung werden die Edelmetalle in Form ihrer Oxide oder Hydroxide ausgefällt. Das Auftränken des oder der Alkalihydroxide und des oder der Alkalimetallsulfate kann getrennt oder gemeinsam erfolgen. Nach einer abschließenden Trocknung steht der Katalysator zur Verfügung.

Er wird in bevorzugter Weise vor seinem Einsatz durch Behandlung mit Wasserstoff bei erhöhter Temperatur, beispielsweise bei 120-400°C, bevorzugt bei 150-380°C, aktiviert, Diese Aktivierung kann mit besonderem Vorteil in dem Reaktor erfolgen, in welchem anschließend die erfindungsgemäße Herstellung von Diphenylamin erfolgt.

Man kann jedoch auch den Träger zunächst mit einer Alkalihydroxidlösung tränken, anschließend trocknen und auf den so vorbehandelten, basisch eingestellten Katalysatorträger die genannten Salze der Edelmetalle auftragen, wobei im Moment der Tränkung auch die Ausfällung der Edelmetalle in Form ihrer Oxide oder Hydroxide erfolgt. Bei dieser Variante kann das zusätzliche Auftränken von Alkalimetallsulfaten gemeinsam mit dem Alkalihydroxid, vor oder nach der Auftragung des Alkalihydroxids oder als abschließende Tränkung nach dem Auftragen der Edelmetalle erfolgen. Auch hier erfolgt nach jedem Auftränken ein separates Trocknen. Nach der anschließenden Trocknung ist auch hierbei wieder der Katalysator einsatzbereit, kann aber in der beschriebenen Weise vorab auch mit Wasserstoff bei erhöhter Temperatur aktiviert werden.

Anstatt den genannten Träger zur Beaufschlagung mit den genannten Stoffen zu tränken, kann er auch mit geeigneten Lösungen besprüht werden. Die zu all diesen Arbeitsvorgängen erforderlichen Geräte und die Einstellung der gewünschten Beaufschlagung durch Wahl der Menge und Konzentration der Lösungen der genannten Elemente sind dem Fachmann im Prinzip bekannt.

Neben wäßrigen Lösungen kommen grundsätzlich auch alkoholische Lösungen oder Lösungen in niederen Carbonsäuren oder niederen Aminen infrage, sofern die vorgesehenen Salze der Edelmetalle beziehungsweise die basischen Alkalimetallverbindungen oder die Sulfate darin löslich sind.

C₁-C₄-Alkyl oder C₁-C₄-Alkoxy in den Substituenten R¹-R⁴ sind beispielsweise Methyl, Ethyl, Propyl, Isopropyl, Butyl, Isobutyl, Methoxy, Ethoxy, Propoxy, Isopropoxy, Butoxy oder Isobutoxy. In bevorzugter Weise haben die genannten Substituenten 1-2 C-Atome, besonders bevorzugt handelt es sich um Methyl beziehungsweise um Methoxy. In weiterhin bevorzugter Weise bedeutet der Substituent R² beziehungsweise R⁴ Wasserstoff, während die Substituenten R¹ und R³ den genannten Bedeutungsumfang annehmen, In besonders bevorzugter Weise richtet sich das Verfahren auf die Herstellung von nicht substituiertem Diphenylamin.

Die Ausgangsverbindungen Anilin und Cyclohexanon beziehungsweise Anilin und Cyclohexanon/Phenol-Gemisch werden im molaren Verhältnis von 1:10-10:1, vorzugsweise 1:2-6:1 eingesetzt. Die Ausgangsverbindungen werden einzeln oder gemeinsam verdampft und das entstehende Dampfgemisch gegebenenfalls mit Hilfe eines Trägergasstromes an den oben beschriebenen Rhodium enthaltenden Katalysator gebracht. Trägergase hierfür sind beispielsweise Stickstoff, Wasserstoff, Argon, niedere Kohlenwasserstoffe, wie Methan, Ethan oder Erdgas sowie Gemische hieraus. In bevorzugter Weise werden Stickstoff oder Wasserstoff oder ein Gemisch von ihnen als Trägergas eingesetzt. Das Trägergas wird in einer Menge von 1-100 l/g Ausgangsmaterial, bevorzugt 1-50 l/g Ausgangsmaterial, eingesetzt. Die Katalysatorbelastung wird mit 0,01-1 kg Ausgangsmaterial je l Katalysator und Stunde festgesetzt.

Neben den genannten Ausgangsverbindungen Anilin und Cyclohexanon und dem bereits beschriebenen teilweisen Ersatz des Cyclohexanons durch das korrespondierende Phenol können weitere Stoffe, wie N-Cyclohexylidenanilin oder Dicyclohexylamin oder N-Cyclohexyl-anilin eingesetzt werden.

Das erfindungsgemäße Verfahren wird bei einer Temperatur von 200-450°C, bevorzugt 250-420°C, und einem Druck von 0,1-20 bar, bevorzugt 1-6 bar, in der Gasphase durchgeführt, Die Kombination von Reaktionstemperatur und Reaktionsdruck werden in einer dem Fachmann bekannten Weise so gewählt, daß stets in Gasphase gearbeitet werden kann.

### Beispiel 1

40 ml (37 g) Rhodiumkatalysator, der gemäß EP 208 933, Beispiel 5, hergestellt worden war, wurde in ein elektrisch beheiztes Rohr von 80 cm Länge und 17 mm Durchmesser gefüllt. Der Katalysator wurde zunächst im Wasserstoffstrom (15-20 l/h) 69 Stunden bei 400°C aktiviert. Bei einer Katalysatortemperatur von 290°C wurde ein Anilin-Cyclohexanon-Gemisch im Mol-Verhältnis 4:1 unter Benutzung einer geeigneten Dosiervorrichtung über den Katalysator geleitet. Als Trägergas wurden 13 l/h Stickstoff verwendet. Im oberen Teil des Reaktionsrohres, in dem sich nur Füllkörper befanden, verdampfte das Gemisch der Ausgangsstoffe und strömte zusammen mit dem Trägergas durch die Katalysatorschicht, die sich im mittleren Abschnitt des Reaktionsrohres befand. Das entstandene Reaktionsprodukt wurde auskondensiert und gaschromatografisch analysiert. Unter stationären Reaktionsbedingungen bei einer Katalysatorbelastung von 0,36 g Gemisch der Ausgangsstoffe/ml Katalysator·h wurde folgendes Reaktionsprodukt erzielt:

| | |
|---|---|
| Diphenylamin | 36,4 % |
| N-Cyclohexyl-anilin | 0,15 % |
| Phenol | 0,4 % |
| Anilin | 62,9 % |
| Nebenprodukte | Rest |

### Beispiel 2

Ein Reaktionsrohr mit einem Durchmesser von 17 mm und einer Länge von etwa 600 mm, dessen oberer Teil als Verdampfungszone diente und das im unteren Teil mit 30 ml eines gemäß DE-OS 3 801 754, Beispiel 2, angefertigten Rh/Pd-Katalysators gefüllt war, wurde durch eine elektrische Beheizung auf 400°C gehalten. Bei dieser Temperatur wurde der Katalysator zunächst 65 Stunden im H₂-Strom aktiviert. Unter Benutzung einer geeichten Dosiervorrichtung wurden innerhalb von 25 Stunden 188 g eines Gemisches aus 4 mol Anilin und 1 mol Cyclohexanon und 10 l N₂/h in das Reaktionsrohr geleitet. Das Reaktionsprodukt wurde kondensiert und gaschromatografisch analysiert. Es ergab sich folgende Zusammensetzung:

| | |
|---|---|
| Diphenylamin | 48,6 % |
| N-Cyclohexyl-anilin | 1,8 % |
| N-Cyclohexyliden-anilin | 0,4 % |
| Anilin | 47,2 % |
| Phenol | 0,4 % |
| Nebenprodukte | Rest |

### Beispiel 3

Zur Umsetzung von Anilin und Cyclohexanon zu Diphenylamin wurde ein Reaktionsrohr mit einem Durchmesser von 32 mm und einer Länge von 80 cm benutzt. Eingesetzt wurden 200 ml (175,4 g) Katalysator, der gemäß DE-OS 3 801 754, Beispiel 2, aus γ-Al₂O₃ in Kugelform (2-6 mm) angefertigt wurde, indem dieser Träger mit 1 % Cr, 1,1 % Mn, 0,5 % Rh, 0,5 % Pt, 2,7 % NaOH und 2,7 % K₂SO₄ imprägniert wurde.

Zunächst wurde der Katalysator 72 Stunden bei 400°C in einem Wasserstoffstrom (60 l/h) aktiviert. Dann wurde bei 315 bis 335°C ein Gemisch von Anilin und Cyclohexanon zusammen mit Stickstoff als Trägergas (12 bis 40 l/h) über den Katalysator geleitet. Das Reaktionsprodukt wurde kondensiert und gaschromatographisch analysiert. Es ergab folgende Zusammensetzung in Abhängigkeit von den Betriebsstunden des Katalysators bei verschiedenen Reaktionsbedingungen (Rest zu 100 % sind Nebenprodukte):

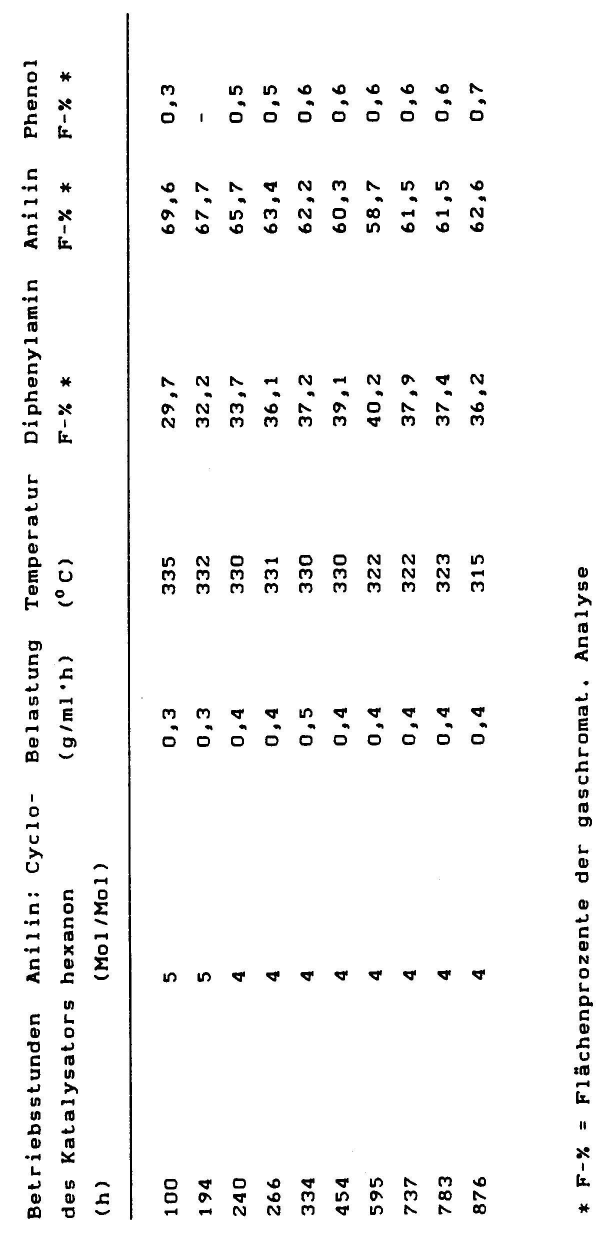

## Patentansprüche

1. Verfahren zur Herstellung von Diphenylaminen der Formel 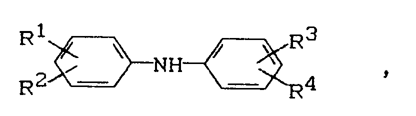 in der
R¹, R², R³ und R⁴ unabhängig voneinander Wasserstoff, C₁-C₄-Alkyl oder C₁-C₄-Alkoxy bedeuten,
dadurch gekennzeichnet, daß Aniline der Formel 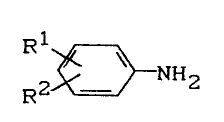 mit Cyclohexanonen der Formel 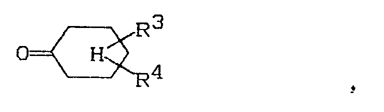 die sich im Gemisch mit recyclisierten Reaktionsprodukten befinden können, wobei in den Formeln die Reste R¹ bis R⁴ die obige Bedeutung haben, und wobei Anilin:Cyclohexanon im molaren Verhältnis von 1:10-10:1, bevorzugt 1:2-6:1, vorliegen, in verdampfter Form an einem 0,05-5 Gew.-%, bevorzugt 0,05-4 Gew.-%, besonders bevorzugt 0,1-3 Gew.-%, Rhodium oder eine Kombination von Rhodium mit mindestens einem Platinmetall aus der Gruppe Palladium, Platin, Ruthenium und Iridium enthaltenden Trägerkatalysator, der weiterhin 1 bis 12 Gew.-%, bezogen auf das Katalysatorgewicht, eines oder mehrerer Alkalimetallhydroxide und/oder Alkalimetallsulfate enthält, wobei die Hydroxidanteile und die Sulfatanteile jeweils höchstens 6 Gew.-% betragen, bei 200-450°C und 0,1-20 bar umgesetzt werden.

2. Verfahren nach Anspruch 1, dadurch gekennzeichnet, daß die Umsetzung bei 250-420°C durchgeführt wird.

3. Verfahren nach Anspruch 1, dadurch gekennzeichnet, daß die Umsetzung bei 0,1-6 bar durchgeführt wird.

4. Verfahren nach Anspruch 1, dadurch gekennzeichnet, daß der Katalysator eine Rhodium/Platinmetall-Kombination enthält, wobei Rhodium 10-90 Gew.-%, bevorzugt 15-80 Gew.-%, besonders bevorzugt 20-70 Gew.-%, an allen Edelmetallen darstellt.

5. Verfahren nach Anspruch 1, dadurch gekennzeichnet, daß der Träger Aluminiumoxid, bevorzugt ein mit Chrom und Mangan behandeltes Aluminiumoxid ist.

6. Verfahren nach Anspruch 1, dadurch gekennzeichnet, daß der Katalysator 2-10 Gew.-%, bezogen auf das Katalysatorgesamtgewicht, eines oder mehrere Alkalimetallhydroxide und/oder Alkalimetallsulfate enthalt, wobei die Hydroxide und Sulfate jeweils höchstens 5 Gew.-% betragen.

7. Verfahren nach Anspruch 1, dadurch gekennzeichnet, daß das Cyclohexanon zu 0-20 mol-% durch das korrespondierende Phenol ersetzt wird.

## Claims

1. Process for the preparation of diphenylamines of the formula 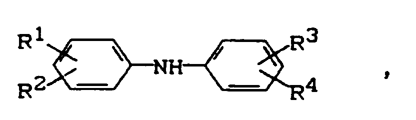 in which
R¹, R², R³ and R⁴, independently of one another, denote hydrogen, C₁-C₄-alkyl or C₁-C₄-alkoxy,
characterised in that anilines of the formula 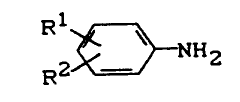 are reacted with cyclohexanones of the formula 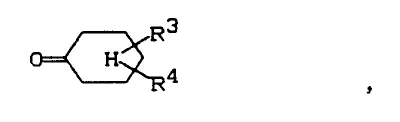 which may be present in a mixture with recycled reaction products, the radicals R¹ - R⁴ in the formulae having the above meaning and aniline and cyclohexanone being present in a molar ratio of 1:10-10:1, preferably 1:2-6:1, in vapourized form over a supported catalyst containing 0.05-5% by weight, preferably 0.05-4% by weight, particularly preferably 0.1-3% by weight, of rhodium or a combination of rhodium with at least one platinum metal from the group consisting of palladium, platinum, ruthenium and iridium, and which furthermore contains 1-12% by weight, relative to the total weight of the catalyst, of one or more alkali metal hydroxides and/or alkali metal sulphates, the hydroxide portions and the sulphate portions each amounting at most to 6% by weight, at 200-450°C and 0.1-20 bar.

2. Process according to Claim 1, characterised in that the reaction is carried out at 250-420°C.

3. Process according to Claim 1, characterised in that the reaction is carried out at 0.1-6 bar.

4. Process according to Claim 1, characterised in that the catalyst contains a rhodium/platinum metal combination in which rhodium represents 10-90% by weight, preferably 15-80% by weight, particularly preferably 20-70% by weight, of all noble metals.

5. Process according to Claim 1, characterised in that the support is alumina, preferably an alumina treated with chromium and manganese.

6. Process according to Claim 1, characterised in that the catalyst additionally contains 2-10% by weight, relative to the total weight of the catalyst, of one or more alkali metal hydroxides and/or alkali metal sulphates, the amounts of hydroxides and sulphates being each at most 5% by weight.

7. Process according to Claim 1, characterised in that 0-20 mol% of the cyclohexanone is replaced by the corresponding phenol.

## Revendications

1. Procédé pour la préparation de diphénylamines répondant à la formule 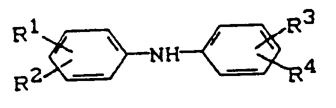 dans laquelle
R¹, R², R³ et R⁴ représentent, indépendamment l'un de l'autre, un atome d'hydrogène, un groupe alkyle en C₁-C₄ ou un groupe alcoxy en C₁-C₄,
caractérisé en ce qu'on fait réagir, à une température de 200-450°C et sous une pression de 0,1-20 bar, des anilines de formule 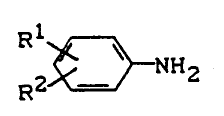 avec des cyclohexanones de formule 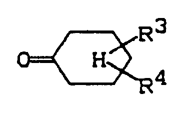 qui peuvent se trouver en mélange avec des produits réactionnels recyclés, dans lequel, dans les formules, les radicaux R¹ à R⁴ possèdent la signification indiquée ci-dessus et dans lequel l'aniline et la cyclohexanone sont présentes dans un rapport molaire de 1:10-10:1, de préférence de 1:2-6:1, sous forme vaporisée sur un catalyseur de support contenant du rhodium ou une combinaison de rhodium avec au moins un métal de platine choisi parmi le groupe comprenant le palladium, le platine, le ruthénium et l'iridium, à concurrence de 0,05-5% en poids, de préférence de 0,05-4% en poids, de manière particulièrement préférée de 0,1-3% en poids, qui contient, en outre, de 1 à 12% en poids, rapportés au poids du catalyseur, d'un ou de plusieurs hydroxydes de métaux alcalins et/ou sulfates de métaux alcalins, les fractions hydroxyde et les fractions sulfate s'élevant respectivement au maximum à 6% en poids.

2. Procédé selon la revendication 1, caractérisé en ce qu'on effectue la mise en réaction à une température de 250-420°C.

3. Procédé selon la revendication 1, caractérisé en ce qu'on effectue la mise en réaction sous une pression de 0,1-6 bar.

4. Procédé selon la revendication 1, caractérisé en ce que le catalyseur contient une combinaison de rhodium/métal de platine dans laquelle le rhodium représente 10-90% en poids, de préférence 15-80% en poids, de manière particulièrement préférée 20-70% en poids de tous les métaux nobles.

5. Procédé selon la revendication 1, caractérisé en ce que le support est de l'oxyde d'aluminium, de préférence un oxyde d'aluminium traité au chrome et au manganèse.

6. Procédé selon la revendication 1, caractérisé en ce que le catalyseur contient 2-10% en poids, rapportés au poids total du catalyseur, d'un ou de plusieurs hydroxydes de métaux alcalins et/ou sulfates de métaux alcalins, les hydroxydes et les sulfates s'élevant respectivement au maximum à 5% en poids.

7. Procédé selon la revendication 1, caractérisé en ce qu'on remplace la cyclohexanone, à concurrence de 0-20 moles %, par le phénol correspondant.
